Europäisches Patentamt

(19) European Patent Office

Office européen des brevets

(11) **EP 0 512 443 B1**

(12) **EUROPÄISCHE PATENTSCHRIFT**

(45) Veröffentlichungstag und Bekanntmachung des Hinweises auf die Patenterteilung:
**10.01.1996 Patentblatt 1996/02**

(51) Int. Cl.$^6$: **A61B 3/08**, G02F 1/137

(21) Anmeldenummer: **92107475.3**

(22) Anmeldetag: **02.05.1992**

(54) **Sehprüfsystem**

Visual testing system

Système de test de vision

(84) Benannte Vertragsstaaten:
**AT CH DE DK FR GB LI NL**

(30) Priorität: **08.05.1991 DE 4115145**

(43) Veröffentlichungstag der Anmeldung:
**11.11.1992 Patentblatt 1992/46**

(73) Patentinhaber:
- **Firma Carl Zeiss**
  **D-73446 Oberkochen (DE)**
  Benannte Vertragsstaaten:
  **CH DE DK FR LI NL AT**
- **CARL-ZEISS-STIFTUNG,**
  **HANDELND ALS CARL ZEISS**
  **D-73446 Oberkochen (DE)**
  Benannte Vertragsstaaten:
  **GB**

(72) Erfinder:
- **Schürle, Hermann**
  **W-7080 Aalen (DE)**
- **Grimm, Wolfgang, Dr.**
  **W-7920 Heidenheim (DE)**
- **Mennicke, Hartmut, Dr.**
  **W-8023 Pullach (DE)**

(56) Entgegenhaltungen:
**EP-A- 0 274 391**       **US-A- 4 097 130**

- **PATENT ABSTRACTS OF JAPAN vol. 10, no. 80 (P-441)(2137) 29. März 1986 & JP-A-60 216 337**
- **COMPUTER TECHNOLOGY REVIEW Bd. VIII, Nr. 13, 1988, LOS ANGELES, USA Seiten 81 - 87; D.BROKENSHIRE ET AL: 'Stereoscopic Display Techniques Improve 3-D Workstation Views'**

**Beschreibung**

Die Erfindung betrifft ein Sehprüfsystem zur Überprüfung der Sehfunktionen eines Probanden, bestehend aus einem Sehprüfgerät und einer Polarisationsbrille, wobei das von dem Sehprüfgerät ausgestrahlte Licht eine jeweils bereichsweise definierte Polarisationsrichtung besitzt und die Polarisationsgläser der Brille eine erste Polarisationsachse für das eine Auge und eine zweite, dazu senkrechtstehende Polarisationsachse für das andere Auge des Probanden besitzen, zum bereichsweisen Sehen des von dem Sehprüfgerät ausgestrahlten Lichtes.

Es sind Sehproben (z.B. EP-PS 0 060 986) und Sehprüfgeräte bekannt, bei welchem ein Prüfling zur Überprüfung der monokularen und binokularen Sehfunktion durch jeweils ein Polarisationsfilter vor jedem Auge mit gegeneinander gekreuzten Schwingungsrichtungen beleuchtete Platten ansieht. Diese Platten haben Bereiche mit unterschiedlicher Polarisationswirkung, so daß der Prüfling mit seinen beiden Augen unterschiedliche Bereiche erkennen kann. Die Sehproben haben den Nachteil, daß die Intensität der Leuchtstärke von der Umgebungsleuchtstärke abhängig ist und daß für jeden Test eine Platte von Hand bewegt werden muß. Sehprüfgeräte vermeiden diese Nachteile, da bei ihnen die Platten von hinten beleuchtet werden und eine Umschaltung auf einen anderen Test durch eine Fernbedienung erfolgen kann, wobei dann mechanisch im Gerät ein Plattenaustausch erfolgt. Die bekannten Sehproben und Sehprüfgeräte haben den gemeinsamen Nachteil, daß die Platten nicht von dem Testenden ohne größere Umstände selber verändert werden können, da die Herstellung der Platten zeitaufwendig ist.

Es ist ein virtuelles stereographisches Bildgebungssystem (US-PS 4 870 486) bekannt, bei welchem vor einer bildgebenden Oberfläche eines TV-Monitors ein Polarisator und eine Flüssigkristallzelle angeordnet ist. Das von diesem Bildgebungssystem ausgehende Licht wird von einem Betrachter durch eine Polaristionsbrille betrachtet, wobei die einzelnen Gläser der Brille eine unterschiedliche Polaristionsachse haben. Dieses Bildgebungssystem ist sehr voluminös wegen des Monitors und dient lediglich der Erzeugung virtueller stereoskopischer Bilder.

Aus der US-PS 4 877 309 ist eine farbig Flüssigkristall-Bilderzeugungseinrichtung bekannt, welche mit zwei Flüssigkristall-Bilderzeugungszellen arbeitet. Vor diesen beiden Bilderzeugungszellen befinden sich jeweils zwei Farbpolarisatoren und an der Lichteinfallsseite der ersten Bilderzeugungszelle befindet sich zusätzlich ein neutraler Polarisator.

Diese farbige Flüssigkristall-Bilderzeugungseinrichtung dient lediglich der Erzeugung farbiger Bilder und ist für sich genommen für ein Sehprüfsystem zur Überprüfung der binokularen Sehfunktionen ungeeignet.

Aus der DE-OS 30 43 511 ist eine Vorrichtung zur Prüfung der Funktionen des Auges und des Sehsystems bekannt. Die Darbietung erfolgt durch Monitore und zwar für die Binokularprüfung mit zwei Monitoren (mit Polaristoren) oder mit einem Monitor (Rot-Grün-Stereopsis). Diese Vorrichtung ist sehr voluminös und bei der Verwendung nur eines Monitors nur sehr begrenzt zur Sehprüfung verwendbar.

Es ist die Aufgabe der Erfindung ein kompaktes Sehprüfsystem zu schaffen, welches die Überprüfung der Sehfunktion mit simultan dargebotenen leicht veränderbaren Sehzeichen ermöglicht. Diese Aufgabe wird durch die Merkmale gemäß dem kennzeichnenden Teil des Anspruchs 1 gelöst.

Das erfindungsgemäße Sehprüfsystem zeichnet sich dadurch aus, daß es trotz seiner Kompaktheit sehr flexibel in seinem Einsatz ist. Wenn man andere Zeichen zur Sehprüfung darstellen will, kann man dies sehr schnell über die Tastatur in den Rechner eingeben. Dieser Rechner sorgt dann über die Ansteuerung der Treiberschaltung der beiden hintereinander angeordneten LCD-Displays LCDI und LCDII dafür, daß das gewünschte Bild auf dem Sehprüfgerät erscheint. Die Ausleuchtung im Sehprüfgerät kann durch eine oder mehrere Lichtquellen erfolgen. Die jeweils einzeln ansteuerbaren Bereiche der beiden LCD-Displays müssen so zueinander ausgerichtet sein, daß die Bereichsgrenzen aufeinanderfallen. Außerdem müssen sie in ihrer Größe zueinanderpassend sein. Das bedeutet nicht, daß sie gleich groß sein müssen. Es ist auch denkbar, daß die LCD-Displays unterschiedlich große, diskret ansteuerbare Flüssigkristallzellen besitzen. Dann legt aber das LCD-Display mit der niedrigeren Auflösung (bzw. geringeren Anzahl von diskret ansteuerbaren Bereichen) die kleinsten darstellbaren Zeichen bzw. Bereiche fest.

Vorteilhafterweise werden Aktiv-Matrix-Displays in TN-Technologie für das Sehprüfsystem verwendet. Diese zeichnen sich durch ein Polarisationsverhalten aus, das nur wenig von der Wellenlänge des verwendeten Lichtes abhängt. Darüberhinaus tritt das bei nicht-aktiven Displays auftretende Übersprechen von angesteuerten Bildpunkten auf nichtangesteuerte Bildpunkte hier nicht auf. Die Aktiv-Matrix-Displays sind über dies sehr schnell im Bildaufbau, was besonders bei bewegten Testbildern vorteilhaft ist.

In vorteilhafter Weise können auch Flüssigkristallanzeigen verwendet werden, die auf dem ferroelektrischen Prinzip arbeiten. Auch diese Zellen verfügen über die Vorteile der Unabhängigkeit von der Wellenlänge des verwendeten Lichtes und des schnellen Bildaufbaus. Dadurch, daß die Bildpunkte bei diesem Display eine Speicherfähigkeit besitzen, bleibt ein einmal eingelesenes Bild auch ohne Ansteuerung weiter bestehen. Dadurch wird die Ansteuerung einfach und ein Übersprechen zwischen den Bildpunkten tritt nicht auf.

Durch die Ansteuerung der beiden LCD-Displays von einer Schalteinrichtung mit einer Tastatur erreicht man ein leichtes Umschalten der durch das Sehprüfgerät erzeugten Zeichen.

Bei der Schalteinrichtung handelt es sich um eine festverdrahtete Schaltung oder einen Rechner bzw. Computer. Verwendet man einen Rechner, so kann man durch einen einfachen Wechsel der Software eine Erzeugung jedes gewünschten Zeichens erreichen.

Unter Tastatur im Sinne der Erfindung ist jede Anordnung zu verstehen, welche geeignet ist, eine eindeutige Auswahl der darzustellenden Zeichen durch die Schalteinrichtung zu ermöglichen. Es kann sich bei der Tastatur aber auch um die normale Tastatur für einen Computer handeln.

Indem man die Lichtquelle in ihrer Intensität von der Schalteinrichtung variiert, kann man eine Aussage über die Sehfähigkeit des Probanden in Abhängigkeit von der Leuchtdichte und dem Kontrast erhalten.

Das nach der Erfindung realisierte Sehprüfgerät kann ohne mechanisch bewegte Teile aufgebaut werden, wodurch es sehr robust, verschleißfrei und störungsunanfällig wird. Außerdem erlaubt es eine sehr leichte Größenvariation der dargestellten Zeichen, wobei die Zeichen jede gewünschte Gestalt besitzen dürfen.

Indem man die Polarisationsachsen der Polarisatoren und der Polaristionsgläser senkrecht zueinander anordnet, erhält man bei leicht geneigter Kopfhaltung des Probanden keine eindeutige Bildtrennung für die beiden Augen. Diese Bildtrennung wird eindeutig bei jeder möglichen Kopfhaltung des Probanden, wenn man vor dem Sehprüfgerät eine Lambda-Viertel-Folie anbringt und die linear polarisierenden Polarisationsgläser der Brille durch circular polarisierende Polarisationsgläser ersetzt. Die Lambda-Viertel-Folien bewirken eine Rotations-Polarisation, welche von der Kopfhaltung des Probanden unabhängig ist.

Sind an der oder den Lichtquellen verschiedene, vorschaltbare Farbfilter oder sind verschiedene Lichtquellen mit unterschiedlichen Farben vorhanden, so kann das Farbsehen des Probanden beurteilt werden.

Ersetzt man das LCD-Display I durch eine farbige Flüssigkristall-Bilderzeugungseinrichtung (z.B. eine Einrichtung gemäß US-PS 4 877 309), so kann man Farbsinnstörungen des Probanden feststellen. Außerdem können dann die Sehzeichen farbig dargestellt werden. Dies ist insbesondere für die Bestimmung von Augengläsern wünschenswert.

Wenn die Prüfsymbole bewegte Sehzeichen sind, kann man weitere Prüfungen der Sehfunktion des Probanden durchführen.

Mit dem nach der Erfindung realisierten Sehprüfgerät läßt sich die "Wendeprobe" bei den nach dem Stand der Technik bekannten Polatest-Geräten erstmals elektronisch realisieren, indem man beim LCD II und/oder LCD I die entsprechenden Pixel des Displays einfach umschaltet.

Die Erfindung wird nachstehend in beispielhafter Weise anhand von drei Zeichnungen näher erläutert, wobei weitere wesentliche Merkmale sowie dem besseren Verständnis dienende Erläuterungen und Ausgestaltungsmöglichkeiten des Erfindungsgedankens beschrieben sind.

Dabei zeigen

Figur 1a-1d     Skizzen zur Erläuterung des Funktionsprinzips;

Figur 2          eine Skizze einer LCD-Display-Oberfläche;

Figur 3          ein erfindungsgemäßes Sehprüfsystem;

Figur 4a       eine Skizze eines Sehprüfsystems mit Farbfilter;

Figur 4b       das Farbfilter in Frontalansicht;

Figur 5          ein weiteres erfindungsgemäßes Sehprüfsystem; und

Figur 6          eine Vereinfachung des Aufbaus des Sehprüfgeräts aus Fig.5.

Anhand der Fig. 1a - d werden die schaltzustände für die vier benötigten verschiedenen Darbietungsarten mittels des erfindungsgemäßen Sehprüfsystems erläutert. Dabei werden die Eigenabsorptionen der Polarisationsfolien und der Flüssigkristallzellen gleich Null gesetzt, da diese in allen zu betrachtenden Fällen eine Konstante darstellen. Bei der Betrachtung wird jeweils nur eine Flüssigkristallzelle jedes Displays berücksichtigt, da für die anderen die gleichen Verhältnisse vorliegen.

Das von der Lichtquelle LQ (1) ausgehende Licht wird durch die erste farbneutrale, durchsichtige Polarisationsfolie Pol I (2) waagrecht polarisiert. Dabei sei angenommen, daß eine Helligkeit von "4 H" durch das erste Polarisationsfilter Pol I (2) gelangt, wobei n mal H von der Helligkeit der Lichtquelle (1) abhängig ist.

In Fig. 1a dreht nun die erste Flüssigkristallzelle LCDI (3) das auf sie auffallende polarisierte Licht um 45°. Dadurch kommt nur noch 2 H durch die zweite senkrecht polarisierende Polarisationsfolie POL II (4). Dreht nun die zweite Flüssigkristallzelle LCDII (5) das auf sie einfallende Licht um 90°, so erreicht das rechte Auge (R) durch die Polarisationsbrille (6) eine Helligkeit von 2 H, während keine Helligkeit das linke Auge (L) durch die Polarisationsbrille (6) erreicht. Die Polarisationsfolien in der Polarisationsbrille (6) sind dabei so ausgerichtet, daß die Polarisationsfolie vor dem linken Auge (L) eine vertikale Polarisationsachse und die Polarisationsfolie vor dem rechten Auge (R) eine waagerechte Polarisationsachse besitzt.

Im Gegensatz zu Fig. 1a dreht die zweite Flüssigkristallzelle LCDII (5) in Fig. 1b das auf sie einfallende Licht um 0°, so daß nun das rechte Auge (R) keine Helligkeit erreicht, während das linke Auge (L) eine Helligkeit von 2 H erreicht.

In Fig. 1c dreht die erste Flüssigkristallzelle LCDI (3) das auf sie einfallende Licht um 90° und die zweite Flüssigkristallzelle LCDII (5) das auf sie einfallende Licht um 45°. Dann erreicht beide Augen (L, R) eine Helligkeit von 2 H.

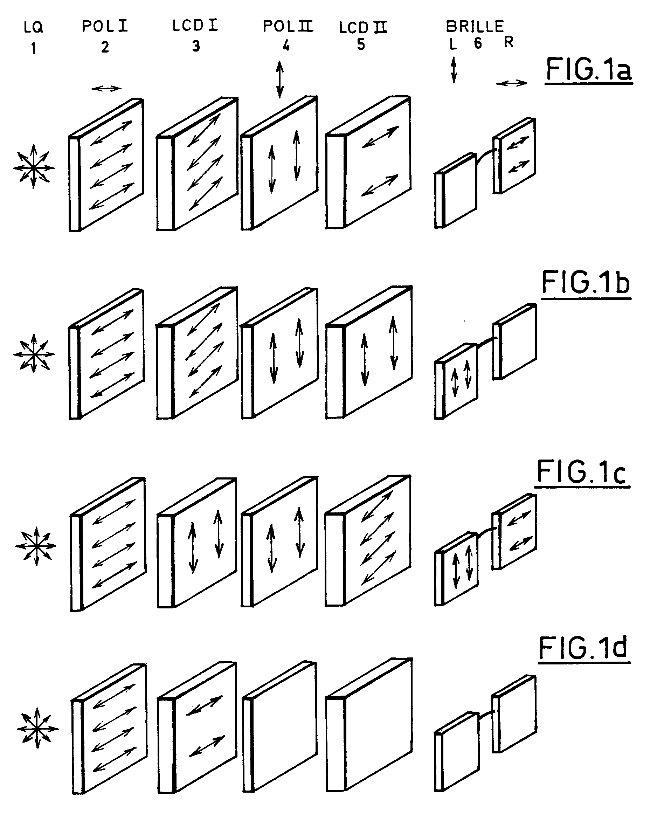

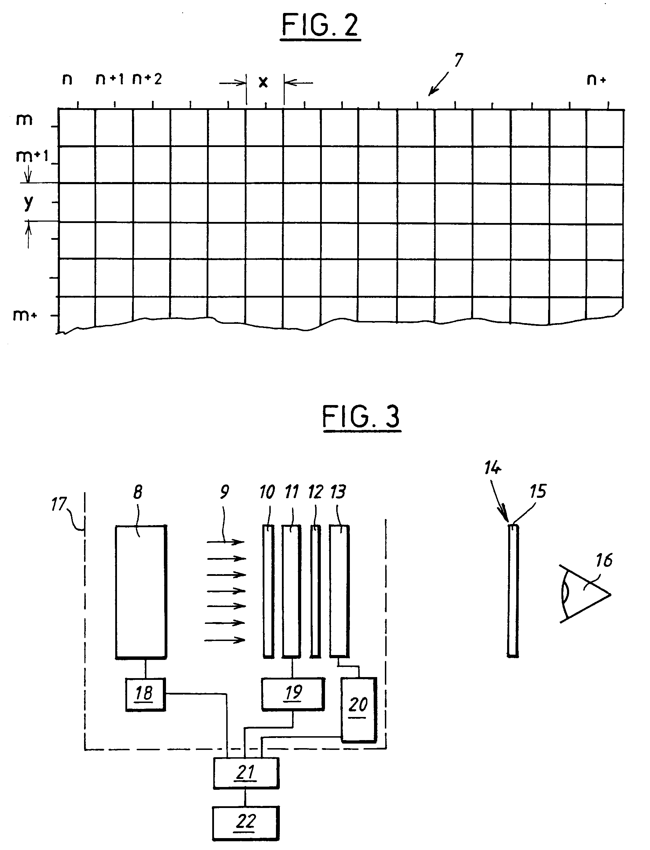

Dreht, wie in Fig. 1d dargestellt, die erste Flüssigkristallzelle LCDI (3) hingegen das auf sie einfallende Licht um 0°, so erreicht keine Helligkeit die beiden Augen (L, R). Setzt man nun mehrere Flüssigkristallzellen aneinander, so erhält man ein LCD-Display (7), wie in Fig. 2 dargestellt. Dabei bezeichnet x die horizontale und y die vertikale Ausdehnung einer Flüssigkristallzelle des Displays (7).

Mit einem derartigen Display (7) kann man nun Figuren und Zeichnungen darstellen, indem man die einzelnen Flüssigkristallzellen stallzellen wie zu Fig. 1a - d erläutert antreibt.

Damit die beiden zur Durchführung der Erfindung benötigten Displays zusammenpassen, müssen die jeweiligen, einzeln ansteuerbaren Flüssigkristallzellen gewisse Bedingungen genügen.

Bezeichnet man bei dem ersten Display die horizontal angeordneten Flüssigkristallzellen mit n (1), n (1) + 1, ..., n (1) + 1 (1) und die vertikal angeordneten Flüssigkristallzellen mit m (1), m (1) + 1, ..., m (1) + k (1), so muß die Anzahl der Flüssigkristallzellen des zweiten Displays (n (2), n (2) + 1, ..., n (2) + 1 (2); m (2), m (2) + 1, ..., m (2) + k (2)) folgende Bedingungen genügen:

$$\sum n\ (1)\ +\ \iota\ (1)\ =\ z \neq \sum n\ (2)\ +\ \iota\ (2)\ \mathrm{mit}\ z \in \mathbb{N} \neq 0\quad n\ (1)\ +\ \iota\ (1) > 2$$

$$\mathrm{oder}$$

$$\sum n\ (1)\ +\ \iota\ (1)\ =\ \frac{1}{z}\ *\ \sum n\ (2)\ +\ \iota\ (2)\ \mathrm{mit}\ z \in \mathbb{N} \neq 0\quad n\ (1)\ +\ 1\ (1) > 2$$

d.h. die Anzahl der Flüssigkristallzellen des zweiten Displays in horizontaler oder vertikaler Richtung darf um ein ganzzahliges Vielfaches voneinander abweichen, ohne daß Randunschärfen bei den darstellbaren Zeichen auftreten müssen. Es ergeben sich aber Auflösungsverluste bei der Darstellung von Zeichen außer bei z = 1.

Bei der Montage müssen die Flüssigkristallzellen der beiden Displays so hintereinander angeordnet werden, daß die Flüssigkristallzellen des ersten Displays einzeln oder in Kombination genau auf eine oder mehrere Flüssigkristallzellen des zweiten Displays fallen, damit unterscheidbare Zeichen darstellbar sind.

Die folgenden Fig. 3, 4a, 4b, 5 und 6 sind Prinzipskizzen, anhand derer unterschiedliche Ausgestaltungsvarianten des Sehprüfsystems erläutert werden sollen.

Für die Prüfung der verschiedenen binokularen und monokularen Sehfunktionen müssen die entsprechenden Sehzeichen unter verschiedenen Bedingungen dargeboten werden.

Zur Prüfung der binokularen Sehfunktionen setzt man dem Proband eine Brille (14) mit je einem Polarisationsglas (15) für jedes Auge (16) auf. Die Polarisationsgläser (15) sind so in die Brille (14) eingesetzt, daß die Polarisationsachsen der beide Polarisationsgläser (15) aufeinander möglichst senkrecht stehen.

Zur Durchführung der Prüfung der binokularen Sehfunktionen muß das Sehprüfgerät (17) mindestens folgende Darbietungsarten realisieren können:

1) Teile eines Sehtestzeichens erscheinen dunkel für ein Auge vor hellem Hintergrund;

2) Teile eines Sehtestzeichens erscheinen hell für ein Auge vor dunklem Hintergrund;

3) unterschiedliche Teile eines Sehtestzeichens erscheinen dunkel für beide Augen vor hellem Hintergrund;

4) unterschiedliche Teile eines Sehtestzeichens erscheinen hell für beide Augen vor dunklem Hintergrund;

(d.h. bei 1) und 2), das die für das eine Auge sichtbaren Teile des Sehzeichens für das andere Auge unsichtbar sind).

Die Prüfung der monokularen Sehfunktionen kann auch ohne Polarisationsbrille (14) erfolgen, wobei mindestens folgende Bedingungen realisiert werden müssen:

5) Sehzeichen erscheint für beide Augen dunkel auf hellem Hintergrund.

6) Sehzeichen erscheint für beide Augen hell auf dunklem Hintergrund.

4

Bei der Prüfung der Farbtüchtigkeit ist es außerdem notwendig, daß folgende Bedingungen realisiert werden können:

7) Teile eines Sehtestzeichens erscheinen unterschiedlich farbig für die beiden Augen vor andersfarbigem Hintergrund;

8a) Sehzeichen erscheint farbig für beide Augen vor hellem Hintergrund;

8b) Sehzeichen erscheint farbig für beide Augen vor dunklem Hintergrund.

Um die Bedingungen 1) - 4) realisieren zu können, muß das erfindungsgemäße Sehprüfgerät (17) wie folgt aufgebaut sein: Eine Lichtquelle (8) beleuchtet mit ihrem Licht (9) eine erste farbneutrale, durchsichtige Polarisationsfolie (10). Das durch die erste Polarisationsfolie (10) fallende Licht durchdringt ein erstes LCD-Display (11) und beleuchtet eine zweite, farbneutrale Polarisationsfolie (12). Das durch die zweite Polarisationsfolie (12) fallende Licht durchdringt ein zweites LCD-Display (13), wonach das Licht das Sehprüfgerät (17) verläßt.

Vor jedem Auge (16) des zu prüfenden Probanden befindet sich ein farbig neutrales, durchsichtiges Polarisationsglas (15). Jede der beiden LCD-Displays (11, 13) ist mit einer eigenen Treiberschaltung (19, 20) verbunden. Die beiden Treiberschaltungen (19, 20) sind mit einer Schalteinrichtung (21) verbunden. Diese Schalteinrichtung (21) ist über eine Tastatur (22) beeinflußbar und steuert die beiden Treiberschaltungen (19, 20) an. Die Schalteinrichtung (21) und die Tastatur (22) kann sich in, bzw. an dem Sehprüfgerät (17) befinden oder über eine gemäß dem bekannten Stand der Technik (z.B. Kabelverbindung, Infrarot- oder Ultraschallverbindung) gestaltete Fernsteuerung eine Umschaltung der Sehzeichen in dem Sehprüfgerät (17) bewirken.

Will man auch die Sehfähigkeit des Probanden in Abhängigkeit von der Leuchtdichte und dem Kontrast prüfen, so wird durch die mit der Lichtquelle (8) verbundene, von der Schalteinrichtung (21) angesteuerte Lichtquellentreiberschaltung (18) zusätzlich die Lichtquelle (8) in ihrer Intensität beeinflußt.

Selbstverständlich kann man mit diesem Sehprüfgerät (17) auch die monokularen und die beidäugigen (ohne Trennung der Seheindrücke des rechten und linken Auges) Sehfunktionen überprüfen, da mit dem Sehprüfgerät (17) auch die Bedingungen 5 und 6 realisiert werden können. Dabei kann der Proband die Polarisationsbrille (14) auflassen oder abnehmen.

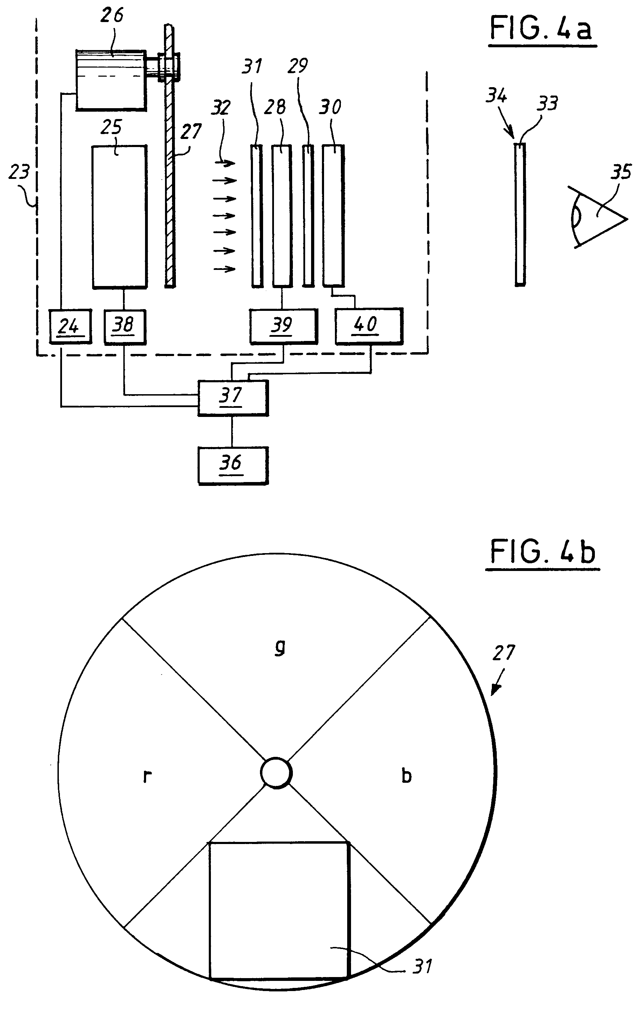

Soll auch eine Überprüfung der Farbtüchtigkeit des Probanden erfolgen, so muß das Sehprüfgerät in der Lage sein, die Sehzeichen und auch Teile davon in Farbe darzustellen. In den folgenden Fig. 4a, 5 und 6 sind Sehprüfsysteme gemäß der Erfindung beschrieben, die dazu in der Lage sind.

In Fig. 4a ist die einfachste Gestaltung eines derartigen, farbtüchtigen Sehprüfgerätes (23) schematisch dargestellt. Es besteht aus zwei Polarisationsfolien (29, 31) und zwei LCD-Displays (28, 30). Die beiden LCD-Displays (28, 30) sind über ihre Treiberschaltungen (39, 40) mit der Schalteinrichtung (37) verbunden, an welcher sich eine Tastatur (36) befindet.

Außer diesen bereits in Fig. 3 beschriebenen Elementen des Sehprüfgerätes (17) ist in diesem Sehprüfgerät (23) eine Farbfilterscheibe (27) vorhanden, welche durch einen Motor (26) gedreht werden kann. Über die Tastatur (36) kann an die Schalteinrichtung (37) die Eingabe erfolgen, daß ein bestimmtes Farbfilter (z.B. rot (r), gelb (g), blau (b), neutral (n); wie in Fig. 4b dargestellt) sich vor der Lichtquelle (25) befinden soll.

In der Schalteinrichtung (37) wird daraufhin die momentane Stellung der Farbfilterscheibe (27) überprüft und wenn sich die Farbfilterscheibe (27) nicht in der gewünschten Position befindet, die Motoransteuerung (24) des Motors (26) der Farbfilterscheibe (27) veranlaßt, die Farbfilterscheibe (27) in die gewünschte Stellung zu drehen. In Fig. 4b ist die Farbfilterscheibe (27) noch einmal in Frontalansicht dargestellt, sowie der Bereich der ersten Polarisationsfolie (31).

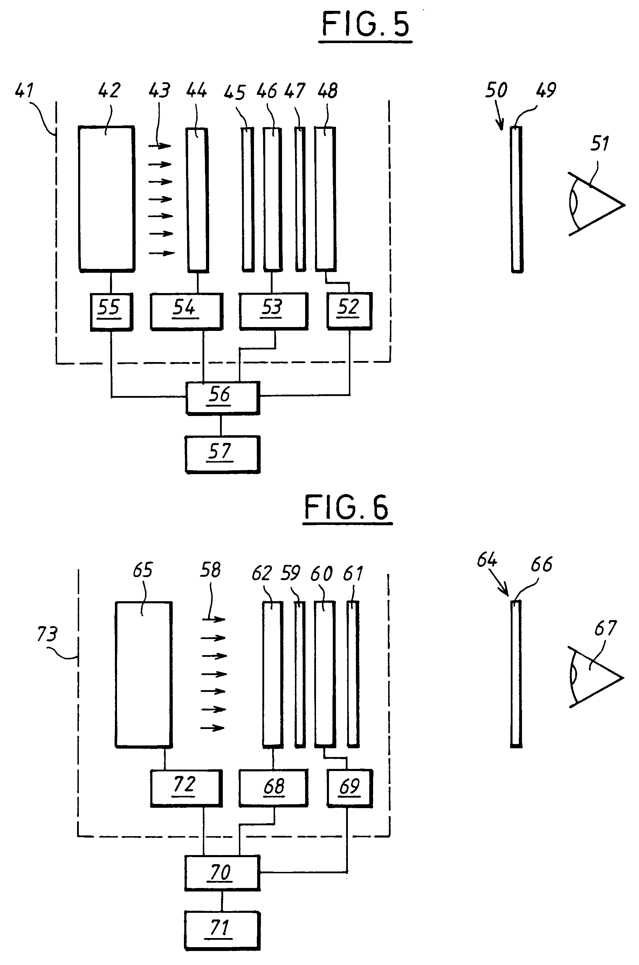

Mit der Farbfilterscheibe (27) aus Fig. 4a und 4b kann man nur die Farbe des ganzen Beleuchtungslichtes (32) verändern. Eine farbige Darstellung mit unterschiedlichen Farben ist nicht möglich. Deshalb ist in Fig. 5 die Farbfilterscheibe (27) mit Motor (26) und Motoransteuerung (24) durch ein Farb-LCD (44) (z.B. farbige Flüssigkristall-Bilderzeugungseinrichtung gemäß der US-PS 4 877 309) mit Ansteuerungsschaltung (54) ersetzt, wobei die Ansteuerungsschaltung (54) mit der Schalteinrichtung (56) verbunden ist.

Das Licht (43) der Lichtquelle (42) gelangt zuerst durch das Farb-LCD (44). Dort wird es in seiner Farbe in gewünschter Art und Weise verändert. Erst dann erfolgt die Erzeugung der Sehzeichen durch die beiden Polarisationsfilter (45, 47) und durch die beiden LCD-Displays (46, 48). Die Lichtquelle (42) kann von der Schalteinrichtung (56) über die Lichtquellentreiberschaltung (55) in ihrer Intensität beeinflußt werden. Die beiden LCD-Displays (46, 48) sind über ihre Treiberschaltungen (52, 53) mit der Schalteinrichtung verbunden.

Die Augen (51) des Probanden können dann durch die Polarisationsbrille (50) mit den beiden Polarisationsfolien (49) ein farbiges Bild sehen.

Die Auflösung dieses Farb-LCD's (44) ist dabei der Auflösung der beiden anderen LCD's (46, 48) gemäß den gemachten Ausführungen zur Auflösung anzupassen.

Mit einem derartig ausgestatteten Sehprüfgerät (41) ist sowohl eine farbige Gestaltung der darstellbaren Sehzeichen als auch eine Darstellung von bewegten Sehzeichen möglich.

Eine Vereinfachung des Aufbaus des Sehprüfgeräts (41) aus Fig. 5 kann man erreichen, wenn man das erste Polarisationsfilter POL I (7) und das erste LCD-Display (8) mit Treiberschaltung (11) ganz durch ein Farb-LCD (62) mit Ansteuerungsschaltung (68) ersetzt (Fig. 6).

Dann dringt das Licht (58) der Lichtquelle (65) zuerst durch das Farb-LCD (62). Das aus dem Farb-LCD (62) austretende Licht mit dem gewünschten Sehzeichen wird dann durch das folgende Polarisationsfilter (59) und das LCD-Display (60) in der gewünschten Art gedreht. In Lichtrichtung nach dem LCD-Display (60) befindet sich eine Lambda-Viertel-Folie (61), welche das durch sie dringende Licht entweder links oder rechts zirkular polarisiert.

In der Brille (64) vor den Augen (67) des Probanden befindet sich auf den Polarisationsfolien nun Lambda-Viertel-Folien (66), so daß die Bedingungen 1 - 4, 5 und 6 als auch die Bedingungen 7, 8a und 8b zur Prüfung der Sehfähigkeit des Probanden realisiert werden können.

Die Verwendung der Lambda-Viertel-Folien auf den Polarisationsfolien (61, 64) hat den Vorteil, daß nun auch bei Schrägstellung des Kopfes des Probanden die Augen des Probanden nur jeweils das sehen, was sie sehen sollen.

Das Sehprüfgerät gemäß der Erfindung zeichnet sich durch seine kompakte, störungsunanfällige Bauweise aus und erlaubt sowohl eine Überprüfung der monokularen und binokularen Sehfunktionen als auch eine Überprüfung der Farbtüchtigkeit des Probanden.

Die beschriebene Polarisationsbrille kann auch als ein aufsteckbarer Aufsatz ausgeführt sein, welche auf eine normale Brille des Probanden zur Überprüfung der Sehfunktionen lediglich aufgesteckt wird.

## Patentansprüche

1. Sehprüfsystem zur Überprüfung der Sehfunktionen eines Probanden, bestehend aus einem Sehprüfgerät und einer Polarisationsbrille, wobei das von dem Sehprüfgerät ausgestrahlte Licht eine jeweils bereichsweise definierte Polarisationsrichtung besitzt und die Polarisationsgläser der Brille eine erste Polarisationsachse für das eine Auge und eine zweite, dazu senkrechtstehende Polarisationsachse für das andere Auge des Probanden besitzen, zum bereichsweisen Sehen des von dem Sehprüfgerät ausgestrahlten Lichtes,
dadurch gekennzeichnet, daß das Sehprüfgerät (17, 23, 41, 63) aus mindestens einer Lichtquelle (8, 25, 42, 65) und hintereinander angeordneten ersten und zweiten LCD-Displays (11, 13; 28, 30; 46, 48) aufgebaut ist, daß an der Lichteinfallsseite jeder der beiden LCD-Displays (11, 13; 28, 30; 46, 48) jeweils ein farbneutraler, durchsichtiger Polarisator (10, 12; 29, 31; 45, 47) angebracht ist, wobei die Polarisationsachse der beiden Polarisatoren (10, 12; 29, 31; 45, 47) unterschiedlich zueinander und jeweils parallel zu den Polarisationsachsen der Polarisationsgläser (15, 33, 49) der Brille (14, 34, 50) ausgerichtet sind, daß jedes LCD-Display (11, 13; 28, 30; 46, 48) durch eine Treiberschaltung (19, 20; 39, 40; 52, 53) diskret ansteuerbar ist, daß die beiden LCD-Displays (11, 13; 28, 30; 46, 48) aus einzeln ansteuerbaren Flüssigkristallzellen aufgebaut sind, welche zueinander ausgerichtet und in ihrer Größe zueinander passend sind, und daß eine Ansteuerung der Treiberschaltung (19, 20; 39, 40; 52, 53) von einer Schalteinrichtung (21, 37, 56) mit einer Tastatur (22, 36, 57) vorhanden ist.

2. Sehprüfsystem nach Anspruch 1, dadurch gekennzeichnet, daß die Lichtquelle (8, 25, 42, 65) in ihrer Intensität von der Schalteinrichtung (21, 37, 56) veränderbar ist.

3. Sehprüfsystem nach einem der Ansprüche 1 oder 2, dadurch gekennzeichnet, daß die Polarisationsachsen der neutralen Polarisatoren (10, 12; 29, 31; 45, 47) zueinander parallel oder senkrecht ausgerichtet sind und daß die Polarisationsachsen der neutralen Polarisatoren (10, 12; 29, 31; 45, 47) und der Polarisationsgläser (15, 33, 49) in zwei zueinander parallel stehenden Ebenen ausgerichtet sind.

4. Sehprüfsystem nach einem der Ansprüche 1 - 3, dadurch gekennzeichnet, daß vor dem Sehpüfgerät eine Lambda-Viertel-Folie (61) angebracht ist und daß die Gläser der Brille circular polarisierende Polarisationsgläser mit Lambda-Viertel-Folien (66) sind.

5. Sehprüfsystem nach einem der Ansprüche 1 - 4, dadurch gekennzeichnet, daß die oder mindestens eine der Lichtquellen (42, 65) in der Farbe des von ihr ausgesendeten Lichtes von der Schalteinrichtung (56, 70) veränderbar ist.

6. Sehprüfsystem nach einem der Ansprüche 1 - 5, dadurch gekennzeichnet, daß vor dem ersten LCD-Display (46) eine farbige Flüssigkristall-Bilderzeugungsvorrichtung (44) vorhanden ist.

7. Sehprüfsystem nach einem der Ansprüche 1 - 5, dadurch gekennzeichnet, daß das erste LCD-Display (11, 28, 46) durch eine farbige Flüssigkristall-Bilderzeugungsvorrichtung (62) ersetzt ist.

**8.** Sehprüfsystem nach einem der Ansprüche 1 - 7, dadurch gekennzeichnet, daß die Flüssigkristallzellen der beiden LCD-Displays so hintereinander angeordnet sind, daß die Flüssigkristallzellen des ersten Displays einzeln oder in Kombination genau auf eine oder mehrere Flüssigkristallzellen des zweiten Displays fallend sind.

**9.** Sehprüfsystem nach einem der Ansprüche 1 - 8, dadurch gekennzeichnet, daß die LCD-Displays Aktiv-Matrix-Displays in TN-Technologie sind.

**10.** Sehprüfsystem nach einem der Ansprüche 1 - 8, dadurch gekennzeichnet, daß die LCD-Displays nach dem ferroelektrischen Prinzip arbeitende Flüssigkeistallzellen sind.

## Claims

**1.** Vision testing system for testing the sight function of a patient, comprising a vision testing apparatus and polarization spectacles, at which the light transmitted by the vision testing apparatus has a defined polarization axis in different regions respectively, the polarization lenses of the spectacle having a first polarization axis for one eye and a second polarization axis perpendicularly to said first polarization axis for the second eye of the patient, the polarization spectacle allow to see the light transmitted by the vision testing apparatus coming from the different regions, characterized in that the vision testing apparatus (17, 23, 41, 63) has at least one light source (8, 25, 42, 65,) and a first and a second LCD-display (11, 13; 28, 30; 46, 48) arranged one after the other, a colour - neutral transparent polarizer (10, 12; 29, 31; 45, 47) mounted on said LCD-displays (11, 13; 28, 30; 46, 48) at the incoming side of the light, at which the polarization axis of the both polarizer (10, 12; 29; 31; 45, 47) have a different orientation respectively and are parallel orientated in respect to the polarization axis of the polarization lenses (15, 33, 49) of the spectacle (14, 34, 50) respectively, each LCD-display (11, 13; 28; 30; 46, 48) has a drive circuit (19, 20; 39, 40; 52, 53) 20; 39, 40; 52, 53) for individually driving, the both LCD-displays (11, 13; 28, 30; 46, 48) include a plurality of individually driveable liquid-crystal cells arranged and sized with respect to each other, and a drive circuit (21, 37, 56) to drive the drive circuits (19, 20, 39, 40; 52, 53) connected with key pad means (22, 36, 57).

**2.** Vision testing system of claim 1, the drive circuit (21, 37, 56) is able to change the intensity of the light of said light source (8, 25, 42, 65).

**3.** Vision testing system of claim 1 or 2, the polarization axis of said neutral polarizer (10, 12; 29, 31; 45, 47) being parallel or perpendicular to each other and the polarization axis of said neutral polarizer (10, 12; 29, 31; 45, 47) and said polarization lenses (15, 33, 49) being arranged in two mutually parallel planes.

**4.** Vision testing system of claim 1, 2 or 3, a /4 filter (61) mounted forward of said vision testing apparatus and an other $\lambda/4$ filter (66) mounted forward of said polarization lenses.

**5.** Vision testing system of claim 1, 2, 3 or 4, the drive circuit (56, 70) is able to change the colour of the light emitted by one ore more of the light sources (42, 65).

**6.** Vision testing system of claim 1, 2, 3, 4 or 5, a colour liquid-Crystal image generating device (44) mounted forward of said first LCD-display (46).

**7.** Vision testing system of claim 1, 2, 3, 4 or 5, wherein said LCD-display (62) is configured as a colour liquid-crystal image generating device (11, 28, 46).

**8.** Vision testing system of claim 1, 2, 3, 4, 5, 6 or 7, the liquid-crystal cells of the both LCD-displays are arranged in the way that the liquid-crystal cells of the first display alone or in combination are arranged exactly in line with one or more liquid-crystal cell of the second display.

**9.** Vision testing system of claim 1, 2, 3, 4, 5, 6, 7 or 8, the LCD-displays are activ-matrix-displays in TN-technology.

**10.** Vision testing system of claim 1, 2, 3, 4, 5, 6, 7 or 8, the LCD-displays have liquid-crystal cells operating on the ferro-electric principle.

## Revendications

**1.** Système de contrôle de la vision d'un sujet, composé d'un appareil de contrôle de la vision et d'une paire de lunettes de polarisation, où la lumière émise par l'appareil de contrôle possède des directions de polarisation definies par zones et où les verres de polarisation de ladite paire de lunettes présentent un premier axe de polarisation pour un oeil du sujet et un second axe de polarisation pour l'autre oeil du client, ce qui permet de voir par zones la lumière émise par ledit appareil de contrôle, caractérisé en ce que l'appareil de contrôle de la vision (17, 23, 41, 63) se compose d'au moins une source de lumière (8, 25, 42, 65) et d'un premier et un second système d'affichage à cristaux liquides (11, 13; 28, 30; 46, 48), qu'un polariseur transparent chromatiquement neutre (10, 12; 29, 31; 45, 47) est aménagé respectivement sur la face d'entrée de lumière de chacun des systèmes d'affichage à cristaux liquides (11, 13; 28, 30; 46, 48), les axes de polarisation des deux polariseurs (10, 12; 29, 31; 45, 47) présentent une orientation différente l'un par rapport à l'autre et une orientation respectivement parallèle aux axes de polarisation des verres de polarisation (15, 33, 49) de la paire de lunettes (14, 34, 50), que chacun des systèmes d'affichage à cristaux liquides (11, 13; 28, 30; 46, 48) peut être commandé séparément par un circuit d'attaque (19, 20; 39, 40; 52, 53), que les deux systèmes d'affichage à cristaux liquides (11, 13; 28, 30; 46, 48) sont composés de cellules à cristaux liquides qui peuvent être activées individuellement et qui sont adaptées les unes aux autres quant à leur orientation et à leur taille, et en ce que le circuit d'attaque (19, 20; 39, 40; 52, 53) peut être commandé à partir d'un organe de commutation (21, 37, 56) doté d'un clavier (22, 36, 57).

2. Système de contrôle de la vision selon la revendication 1, caractérisé en ce que l'intensité de la source de lumière (8, 25, 42, 65) peut être réglée à partir de l'organe de commutation (21, 37, 56).

3. Système de contrôle de la vision selon l'une des revendications 1 et 2, caractérisé en ce que les axes de polarisation des polariseurs neutres (10, 12; 29, 31; 45, 47) sont orientés parallèlement ou perpendiculairement l'un par rapport à l'autre et que les axes de polarisation des polariseurs neutres (10, 12; 29, 31; 45, 47) et ceux des verres de polarisation (15, 33, 49) évoluent dans deux plans parallèles l'un à l'autre.

4. Système de contrôle de la vision selon l'une des revendications 1 à 3, caractérisé en ce qu'une teinte sensible d'un quart de lambda (61) est montée en amont de l'appareil de contrôle de la vision et que les verres de la paire de lunettes sont des verres circulairement polarisants dotés de teintes sensibles d'un quart de lambda (66).

5. Système de contrôle de la vision selon l'une des revendications 1 à 4, caractérisé en ce que la source de lumière ou qu'une au moins des sources de lumière (42, 65) émet une lumière dont la couleur peut être modifiée par l'organe de commutation (56, 70).

6. Système de contrôle de la vision selon l'une des revendications 1 à 5, caractérisé en ce qu'un dispositif de formation d'images couleurs à cristaux liquides (44) est aménagé devant le premier système d'affichage à cristaux liquides (46).

7. Système de contrôle de la vision selon l'une des revendications 1 à 5, caractérisé en ce que le premier système d'affichage à cristaux liquides (11, 28, 46) est remplacé par un dispositif de formation d'images couleurs à cristaux liquides (62).

8. Système de contrôle de la vision selon l'une des revendications 1 à 7, caractérisé en ce que les cellules à cristaux liquides des deux systèmes d'affichage sont disposées les unes derrières les autres, de manière à ce que les cellules du premier système d'affichage sont reproduites, individuellement ou en combinaison, sur une ou plusieurs des cellules à cristaux liquides du second système d'affichage.

9. Système de contrôle de la vision selon l'une des revendications 1 à 8, caractérisé en ce que les systèmes d'affichage à cristaux liquides sont des systèmes à matrice active fabriqués selon la technologie TN.

10. Système de contrôle de la vision selon l'une des revendications 1 à 8, caractérisé en ce que les systèmes d'affichage sont composés de cellules à cristaux liquides fonctionnant selon le principe ferro-électrique.

FIG.1a

FIG.1b

FIG.1c

FIG.1d

## FIG. 2

## FIG. 3

FIG. 4a

FIG. 4b

EP 0 512 443 B1

## FIG. 5

## FIG. 6

12